(19) Europäisches Patentamt / European Patent Office / Office européen des brevets

(11) **EP 3 950 719 A1**

(12) **EUROPEAN PATENT APPLICATION**
published in accordance with Art. 153(4) EPC

(43) Date of publication:
**09.02.2022 Bulletin 2022/06**

(51) International Patent Classification (IPC):
*C07K 19/00* (2006.01)    *C12N 15/11* (2006.01)
*C12N 15/63* (2006.01)    *C12N 15/00* (2006.01)

(21) Application number: **20772665.4**

(52) Cooperative Patent Classification (CPC):
**C07K 19/00; C12N 15/00; C12N 15/11;**
**C12N 15/62; C12N 15/63; C12N 15/70**

(22) Date of filing: **18.03.2020**

(86) International application number:
**PCT/CN2020/080036**

(87) International publication number:
**WO 2020/187270 (24.09.2020 Gazette 2020/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.03.2019  CN 201910210102**

(71) Applicant: **Ningbo Kunpeng Biotech Co., Ltd.
Yuyao Ningbo, Zhejiang 315400 (CN)**

(72) Inventors:
• **WU, Song**
  **Ningbo, Zhejiang 315400 (CN)**
• **ZHANG, Zhenshan**
  **Ningbo, Zhejiang 315400 (CN)**
• **LIU, Huiling**
  **Ningbo, Zhejiang 315400 (CN)**
• **CHEN, Wei**
  **Ningbo, Zhejiang 315400 (CN)**

(74) Representative: **Lavoix
Bayerstraße 83
80335 München (DE)**

(54) **FUSION PROTEIN CONTAINING FLUORESCENT PROTEIN FRAGMENTS AND USES THEREOF**

(57)    Provided are a fusion protein containing fluorescent protein fragments and uses thereof. The fusion protein according to the present invention has a structure as expressed by (P1-L1)s-A1-(X)n-A2-(L2-P2)t, wherein (X)n or A1-(X)n can promote the folding and expression of fused target peptide, improve the solubility of the fusion peptide and reduce the interaction among fusion peptide molecules. (X)n or A1-(X)n in the fusion protein can be cut by enzymes into a plurality of short peptides with a length much shorter than that of the target peptide, which facilitates the separation from the target peptide so as to make the purification of the target peptide more convenient.

EP 3 950 719 A1

**Description**

**Technical field**

The present invention relates to the field of biotechnology, in particular to fusion proteins containing fluorescent protein fragments and uses thereof.

**Background**

Peptides are a class of biomolecules, which are widely used as reagents in many biomedical research fields, as therapeutic drugs in the treatment of diseases, as diagnostic agents in the detection of pathogenic bacteria, and as biomarkers. There are usually two methods to synthesize peptides, one is chemical synthesis, and the other is recombinant expression. Chemical synthesis has been used to prepare a variety of therapeutic peptides, including corticorelin, parathyroid hormone (PTH), glucagon-like peptide (GLP-1) and its derivatives exenatide and liraglutide, enfuvirtide, calcitonin, bivalirudin, ziconotide, semorelin, somatorelin, secretin, teduglutide, and insulin. This method requires multi-step condensation of amino acid fragments to form peptides, and also requires cumbersome protection, deprotection, and purification steps. So far, most commercial peptides with less than 50 amino acid residues are produced by this method. As the demand for peptides in the pharmaceutical industry and biomedical research continues to increase, the price of amino acid fragments for chemical synthesis is also rising. Therefore, the daily use of therapeutic peptide drugs such as GLP-1 analogs will be difficult to maintain affordable prices in the future. Although it is technically feasible to chemically synthesize peptides with less than 50 amino acid residues, the lower yield and the large amount of organic waste generated during the synthesis process are quite uneconomical. At present, most peptides with more than 50 amino acid residues are recombinantly expressed in cell hosts such as bacteria, yeast, insects, and mammals. For many years, the use of fusion proteins to express polypeptides has been a common method. However, the currently available fusion protein methods for peptide expression have many technical problems, especially for peptides that produce less than 50 amino acid residues. For example, the fusion protein in the prior art has a large molecular weight, strong hydrophobicity, and is difficult to separate. The target protein has a low specific gravity, low fusion ratio, stable structure, difficult to digest, and has a lot of dead volume in the loading ion column and the hydrophobic column.

Therefore, it is very necessary to develop a new fusion protein for expressing the target peptide, to overcome the limitations of existing fusion proteins, and at the same time to increase the yield of non-natural amino acid proteins inserted, such as increasing the production of non-natural amino acid proteins such as BOC-lysine proteins.

**Summary of the invention**

The purpose of the present invention is to provide a new fusion protein for expressing a target peptide, and at the same time, it can increase the yield of non-natural amino acid proteins inserted, such as increasing the yield of non-natural amino acid proteins such as BOC-lysine proteins.

In a first aspect of the present invention, it provides a fusion protein, comprising the structure as shown in Formula I:

$$(P1\text{-}L1)_s\text{-}A1\text{-}(X)_n\text{-}A2\text{-}(L2\text{-}P2)_t \quad (I)$$

wherein

"-" is a peptide bond or linker peptide,
each P1 is independently a first target peptide;
each P2 is independently a second target peptide;
each L1 is independently none or a first linker peptide;
each L2 is independently none or a second linker peptide;
A1 is none or signal peptide;
A2 is none or signal peptide;
each X is independently a single β-folding unit of fluorescent protein;
n is a positive integer from 1-8;
s is 0, 1, or 2;
t is 0, 1, or 2; and
the additional condition is that s and t are not 0 at the same time.

In another preferred embodiment, s is 0 and t is 1.

In another preferred embodiment, s is 1 and t is 0.

In another preferred embodiment, the n is 1-6, preferably, n is 2-4.

In another preferred embodiment, the A1 is a signal peptide, and A2 is none.

In another preferred embodiment, the β-folding unit is selected from the group consisting of the β-folding units u1, u2, u3, u4, u5, u6, u7, u8, u9, u10 and u11 of the fluorescent protein.

In another preferred embodiment, the length of each X is 10-14 aa.

In another preferred embodiment, each X is different.

In another preferred embodiment, each X is the same.

In another preferred embodiment, the fluorescent protein is selected from the group consisting of green fluorescent protein (GFP), yellow fluorescent protein (YFP), blue fluorescent protein (BFP), cyan fluorescent protein gene (CFP), and a combination thereof.

In another preferred embodiment, the fluorescent protein is green fluorescent protein (GFP).

In another preferred embodiment, the GFP has an amino acid sequence as shown in SEQ ID NO.: 13.

In another preferred embodiment, there is or does not have a flexible joint I between any two β-folding units.

In another preferred embodiment, the (X)n serves as a protein expression promoting element.

In another preferred embodiment, the total length Ln of (X)n is 3.7-30% of the total length L0 of the fluorescent protein, preferably 7.5-20.5%, more preferably 7.5-15.5%.

In another preferred embodiment, the X is selected from the group consisting of:

uj, which has an amino acid sequence as shown in SEQ ID NO.: j;
wherein, j is 1, 2, 3, 4, 5, 6, 7, 8, 9, 10, or 11.

In another preferred embodiment, the X is selected from the group consisting of:

| X | Amino acid sequence |
|---|---|
| u1 | VPILVELDGDVNG (SEQ ID NO.: 1) |
| u2 | HKFSVRGEGEGDAT (SEQ ID NO.: 2) |
| u3 | KLTLKFICTT (SEQ ID NO.: 3) |
| u4 | YVQERTISFKD (SEQ ID NO.: 4) |
| u5 | TYKTRAEVKFEGD (SEQ ID NO.: 5) |
| u6 | TLVNRIELKGIDF (SEQ ID NO.: 6) |
| u7 | HNVYITADKQ (SEQ ID NO.: 7) |
| u8 | GIKANFKIRHNVED (SEQ ID NO.: 8) |
| u9 | VQLADHYQQNTPIG (SEQ ID NO.: 9) |
| u10 | HYLSTQSVLSKD (SEQ ID NO.: 10) |
| u11 | HMVLLEFVTAAGI (SEQ ID NO.: 11) |

In another preferred embodiment, the X further includes an amino acid sequence generated by mutual substitution of R, K, and H in any of the sequences as shown in SEQ ID NO.: 1-11; and/or

The X further includes an amino acid sequence formed by mutual substitution of P and Q in the sequence as shown in any one of SEQ ID NO.: 1-11; and/or

The X further includes an amino acid sequence formed by mutual substitution of T and S in the sequence as shown in any one of SEQ ID NO.: 1-11.

In another preferred embodiment, the X is selected from the group consisting of:

(A) a polypeptide having the amino acid sequence as shown in any one of SEQ ID NO: 1-11;

(B) a polypeptide having ≥80% homology (preferably ≥85%, more preferably ≥90%, more preferably ≥95%, most preferably ≥97% homology) with the amino acid sequence as shown in any one of SEQ ID NO: 1-11 and retaining the characteristics;

(C) a derivative polypeptide formed by substituting, deleting or adding 1-5 amino acid residues to the amino acid sequence as shown in any one of SEQ ID NO: 1-11, and retaining the characteristics.

In another preferred embodiment, the (X)n is u8, u9, u2-u3, u4-u5, u8-u9, u1-u2-u3, u2-u3-u4, u3-u4-u5, u5-u6-u7, u8-u9-u10, u9-u10-u11, u3-u5-u7, u3-u4-u6, u4-u7-u10, u6-u8-u10, u1-u2-u3-u4, u2-u3-u4-u5, u3-u4-u3-u4, u3-u5-u7-u9, u5-u6-u7-u8, u1-u3-u7-u9, u2-u2-u7-u8, u7-u2-u5-u11, u3-u4-u7-u10, u1-l-u2, u1-l-u5, u2-l-u4, u3-l-u8, u5-l-u6, or u10-l-u11.

In another preferred embodiment, the A1 or A2 has an amino acid sequence as shown in SEQ ID NO.: 12 (MVSKGEELFTGV).

In another preferred embodiment, the A1-(X)n has an amino acid sequence as shown in SEQ ID NO.: 14, 15, 16, 17, 22, 23, 24, 26, 27, 28, 29 or 30 .

In another preferred embodiment, the first linker peptide contains a first restriction site (such as a TEV restriction site).

In another preferred embodiment, the second linker peptide contains a second restriction site.

In another preferred embodiment, the flexible linker I contains a third restriction site.

In another preferred embodiment, the third restriction site is an EK restriction site (as shown in the sequence DDDDK, SEQ ID NO.: 25).

In another preferred embodiment, the (X)n is u1-EK-u2, u1-EK-u5, u2-EK-u4, u3-EK-u8, u5-EK-u6, or u10-EK-u11, wherein EK is restriction site of EK enzyme.

In another preferred embodiment, the first, second and third restriction sites are different from each other.

In another preferred embodiment, two or three of the first, second and third restriction sites are the same.

In another preferred embodiment, the first, second and third restriction sites are not present in P1 and P2.

In another preferred embodiment, the first linker peptide and/or the second linker peptide further contain restriction sites different from the first, second and third restriction sites.

In another preferred embodiment, the first linker peptide and/or the second linker peptide contain a trypsin restriction site, preferably, the first linker peptide and/or the second linker peptide contain at least one Arginine (R) or Lysine (K).

In another preferred embodiment, the N-terminal amino acid of the first linker peptide is Arg or Lys.

In another preferred embodiment, the C-terminal amino acid of the second linker peptide is Arg or Lys.

In another preferred embodiment, the first linker peptide and/or the second linker peptide further contain a tobacco etch virus protease recognition sequence.

In another preferred embodiment, the first linker peptide and/or the second linker peptide have an amino acid sequence as shown in SEQ ID NO.: 18.

In another preferred embodiment, the first linker peptide and/or the second linker peptide further contain a tag sequence that assists in expression and/or purification.

In another preferred embodiment, the tag sequence is a histidine tag, preferably a 6×HIS tag.

In another preferred embodiment, the P1 and P2 are each independently selected from: human insulin precursor protein, insulin lispro precursor protein, insulin glargine precursor protein, parathyroid hormone, corticorelin, calcitonin, bivalirudin, glucagon-like peptide and its derivatives exenatide and liraglutide, ziconotide, sermorelin, comatorelin, secretin, teduglutide, hirudin, growth hormone, growth factor, growth hormone releasing factor, adrenocorticotrophic hormone, releasing factor, deslorelin, desmopressin, elcatonin, glucagon, leuprorelin, luteinizing hormone releasing hormone, somatostatyna, thyrotropin-releasing hormone, triptorelin, vasoactive intestinal peptide, interferon, parathyroid hormone, BH3 peptide, amyloid peptide, or fragments of the above peptides, and a combination thereof, preferably but not limited to the above-mentioned polypeptides.

In another preferred embodiment, the P1 and P2 are each independently a protein with an unnatural amino acid.

In another preferred embodiment, the P1 and P2 each independently have a sequence of 10-200 amino acids in length, preferably, a sequence of 10-80 amino acids in length.

In another preferred embodiment, the P1 and P2 are each independently proinsulin or insulin, preferably human proinsulin or human insulin.

In another preferred embodiment, the insulin includes long-acting or fast-acting insulin.

In another preferred embodiment, the lysine at position 29 of the proinsulin is an alkynyloxycarbonyl lysine derivative, a BOC-lysine (tert-butoxycarbonyl-L-lysine) derivative , Fatty acylated lysine, and a combination thereof.

In another preferred embodiment, the proinsulin has the amino acid sequence as shown in SEQ ID NO: 19 or 20.

In another preferred embodiment, the target peptide P2 is located at (or connected to) the C-terminus of (X)n.

In another preferred embodiment, the fusion protein is digested to form the first target peptide and/or the second target peptide.

In another preferred embodiment, the first target peptide and the second target peptide are the same or different.

In another preferred embodiment, the fusion protein is digested to form the first target peptide and/or the second target peptide, and the (X)n is cut into a short peptide with a length Lx much smaller than the length Lp of the first target peptide and/or the second target peptide.

In another preferred embodiment, each Lx is 1-25 amino acids.

In another preferred embodiment, the ratio of the length Lx to the length Lp is 1/2 to 1/10, preferably 1/3 to 1/8.

In another preferred embodiment, the difference between the length Lp and the length Lx is greater than 1.3KD.

In another preferred embodiment, the fusion protein has a structure selected from the group consisting of: A1-u8-L2-P2, A1-u9-L2-P2, A1-u2-u3-L2-P2, A1-u4-u5-L2-P2, A1-u8-u9-L2-P2, A1-u3-u5-u7-L2-P2, A1-u1-u2-u3-L2-P2, A1-u1-u2-u3-u4-L2-P2, A1-u3-u4-u6-L2-P2, A1-u4-u7-u10-L2-P2, A1-u3-u4-u7-u10-L2-P2, or A1-u5-EK-u6 -L2-P2.

In another preferred embodiment, the fusion protein has an amino acid sequence as shown in SEQ ID NO.: 21.

In a second aspect of the present invention, it provides an isolated polynucleotide encoding the fusion protein according to the first aspect of the present invention.

In a third aspect of the present invention, it provides a vector, which includes the polynucleotide according to the second aspect of the present invention.

In another preferred embodiment, the vector is selected from the group consisting of DNA, RNA, plasmid, lentiviral vector, adenoviral vector, retroviral vector, transposon, and a combination thereof.

In another preferred embodiment, the vector is a plasmid, preferably, the vector is a pBAD-HisA vector and/or a pEvol-pBpF vector.

In a fourth aspect of the present invention, it provides a host cell containing the vector according to the third aspect of the present invention, or the polynucleotide according to the second aspect of the present invention integrated into the chromosome, or expresses the fusion protein according to the first aspect of the present invention.

In another preferred embodiment, the host cell is Escherichia coli, Bacillus subtilis, a yeast cell, an insect cell, a mammalian cell and a combination thereof.

In another preferred embodiment, none of the host cell contains the proteases corresponding to the first, second and third restriction sites.

In a fifth aspect of the present invention, it provides a method for preparing a protein, comprising the steps:

(a) culturing the host cell according to the fourth aspect of the present invention, thereby obtaining the fusion protein according to the first aspect of the present invention.

In another preferred embodiment, the method further comprises the step of: (b) purifying the fusion protein obtained in step (a).

In another preferred embodiment, the method further comprises performing proteolytic digestion of the fusion protein according to the first aspect of the present invention to release the target peptide from the fusion protein.

In another preferred embodiment, in the step (c), digesting the fusion protein according to the first aspect of the present invention with a protease to obtain a digested product; and optionally
(d) isolating or purifying the target peptide from the digested product.

In another preferred embodiment, the purification includes further purification of the target peptide by gel filtration or HPLC.

In a sixth aspect of the present invention, it provides use of the fusion protein according to the first aspect of the present invention, or the polynucleotide according to the second aspect of the present invention, or the vector according to the third aspect of the present invention, or the host cell according to the fourth aspect of the present invention for expression and preparation of the target peptide.

It should be understood that within the scope of the present invention, the above-mentioned technical features of the present invention and the technical features described in detail below (such as embodiments) can be combined with each other to form a new or preferred technical solution. Due to space constraints, I will not repeat them here.

## Description of the drawings

Figure 1 shows the structure of mature insulin that correctly forms disulfide bonds.

Figure 2 shows the recombinant human insulin fusion protein expression plasmid map.

Figure 3 shows a schematic diagram of recombinant human insulin fusion protein.

Figure 4 shows the structure of BOC-Lysine.

Figure 5 shows the recombinant fusion protein expression. Lane 1, Expression of A1-u4-u5-TEV-R-MiniINS fusion protein, with a molecular weight of 11.2kD; Lane 2, Expression of A1-u4-u5-TEV-R-GLP1 fusion protein, with a molecular weight of 8.7kD; Lane 3, Expression of A1-u8-TEV-R-MiniINS fusion protein, with a molecular weight of 10.0kD; lane 4, expression of A1-u3-u5-u7-TEV-R-MiniINS fusion protein, with a molecular weight of 12.2kD; lane 5, Expression of A1-u5-EK-u6-TEV-R-MiniINS fusion protein, with a molecular weight of 12.0kD; M is the protein molecular weight standard.

## DETAILED DESCRIPTION

After extensive and in-depth research, the inventors have obtained a new fusion protein suitable for expressing the target peptide for the first time. The (X)n or A1-(X)n in the fusion protein of the present invention can promote the folding and expression of the fusion target peptide, and can increase the yield and solubility of the fusion protein and reduce the intermolecular interaction of the fusion protein, so that the fusion target peptide can be folded at a high concentration of commercial significances. In addition, the (X)n or A1-(X)n in the fusion protein can be cleaved into multiple short peptides whose length is much smaller than the length of the target peptide, which is more conducive to its separation from the target peptide, thereby making the purification of the target peptide more convenient. On this basis, the inventors have completed the present invention.

**Terms**

Before describing the present invention, it should be understood that the present invention is not limited to the specific methods and experimental conditions as described, because such methods and conditions can vary. It should also be understood that the terms used herein are only intended to describe specific embodiments and are not intended to be limiting, and the scope of the present invention will only be limited by the appended claims.

Unless otherwise defined, all technical and scientific terms used herein have the same meaning as commonly understood by those of ordinary skill in the art to which the present invention belongs.

As used herein, when used in reference to a specifically recited value, the term "about" means that the value can vary from the recited value by no more than 1%. For example, as used herein, the expression "about 100" includes all values between 99 and 101 (e.g., 99.1, 99.2, 99.3, 99.4, etc.).

As used herein, the term "containing" or "comprising (including)" can be open, semi-closed, and closed. In other words, the term also includes "substantially consisting of" or "consisting of".

The three-letter codes and one-letter codes of amino acids used in the present invention are as described in J. biool. chem, 243, p3558 (1968).

As used herein, the term "optional" or "optionally" means that the event or situation described later can occur but does not have to occur.

The "sequence identity" in the present invention refers to the degree of identity between two nucleic acid or two amino acid sequences when optimally aligned and compared with appropriate mutations such as substitutions, insertions, or deletions. The sequence identity between the sequence described in the present invention and its identical sequence may be at least 85%, 90% or 95%, preferably at least 95%. Non-limiting examples include 85%, 86%, 87%, 88%, 89%, 90%, 91%, 92%, 93%, 94%, 95%, 96%, 97%, 98%, 99%, 100%.

**Fusion protein**

As used herein, "fusion protein of the present invention", "recombinant fusion protein" or "polypeptide" all refer to the fusion protein according to the first aspect of the present invention.

Specifically, the fusion protein of the present invention includes the structure as shown in Formula I:

$$(P1-L1)s-A1-(X)n-A2-(L2-P2)t \ (I)$$

wherein

"-" is a peptide bond or linker peptide,
each P1 is independently a first target peptide;
each P2 is independently a second target peptide;
each L1 is independently none or a first linker peptide;
each L2 is independently none or a second linker peptide;
A1 is none or signal peptide;
A2 is none or signal peptide;
each X is independently a single $\beta$-folding unit of fluorescent protein;
n is a positive integer from 1-8;
s is 0, 1, or 2;
t is 0, 1, or 2; and
the additional condition is that s and t are not 0 at the same time.

In another preferred embodiment, the fluorescent protein is GFP, preferably, the amino acid sequence of the GFP has the amino acid sequence (241 AA) as shown in SEQ ID NO.: 13.

MVSKGEELFTGV<u>VPILVELDGDV</u>NG<u>HKFSVRGEGEGD</u>ATNG<u>KLTLKFIC</u>
<u>TTGKLPVPWPTLVTTLTYGVQCFSRYPDHMKRHDFFKSAMPEG</u><u>YVQERTISF</u>
<u>KDDGT</u><u>YKTRAEVKFEGD</u><u>TLVNRIELKGI</u>DFKEDGNILGHKLEYNFNS<u>HNVYI</u>
<u>TAD</u>KQKN<u>GIKANFKIRHNV</u>EDGS<u>VQLADHYQQNTP</u>IGDGPVLLPDN<u>HYLST</u>
<u>QSVLSKDPNEKRD</u><u>HMVLLEFVTAA</u>GITHGMDELYAGS (SEQ ID NO.: 13)

Among them, in the above sequence, the underlined parts are the β-folding units u1, u2, u3, u4, u5, u6, u7, u8, u9, u10, and u11 of the fluorescent protein in sequence.

In another preferred embodiment, the (X)n is u8, u9, u2-u3, u4-u5, u8-u9, u1-u2-u3, u2-u3-u4, u3-u4-u5, u5-u6-u7, u8-u9-u10, u9-u10-u11, u3-u5-u7, u3-u4-u6, u4-u7-u10, u6-u8-u10, u1-u2-u3-u4, u2-u3-u4-u5, u3-u4-u3-u4, u3-u5-u7-u9, u5-u6-u7-u8, u1-u3-u7-u9, u2-u2-u7-u8, u7-u2-u5-u11, u3-u4-u7-u10, u1-I-u2, u1-I-u5, u2-I-u4, u3-I-u8, u5-I-u6, or u10-I-u11.

In another preferred embodiment, each X(uj) in the (X)n has or does not have a flexible joint I, preferably a flexible linker I.

In another preferred embodiment, the flexible linker I contains a third restriction site.

In another preferred embodiment, the flexible linker I contains restriction site of EK enzyme (DDDDK).

In another preferred embodiment, the fusion protein is digested to form a first target peptide and/or a second target peptide, and the (X)n is cut into a short peptide with a length Lx much smaller than the length Lp of a first target peptide and/or a second target peptide.

In another preferred embodiment, the A1-(X)n is A1-u8, A1-u9, A1-u2-u3, A1-u4-u5, A1-u8-u9, A1-u3-u5-u7, A1-u1-u2-u3, A1-u1-u2-u3-u4, A1-u3-u4-u6, A1-u4-u7-u10, A1-u3-u4-u7-u10, or A1-u5-EK-u6.

In another preferred embodiment, the A1-(X)n has an amino acid sequence as shown in SEQ ID NO.: 14, 15, 16, 17, 22, 23, 24, 26, 27, 28, 29 or 30 .

A1-u8 sequence:
MVSKGEELFTGVGIKANFKIRHNVED (SEQ ID NO.: 23)
A1-u9 sequence:
MVSKGEELFTGVVQLADHYQQNTPIG (SEQ ID NO.: 17)
A1-u2-u3 sequence:
MVSKGEELFTGVHKFSVRGEGEGDATKLTLKFICTT (SEQ ID NO.: 24)
A1-u4-u5 sequence:
MVSKGEELFTGVYVQERTISFKDTYKTRAEVKFEGD (SEQ ID NO.: 16)
A1-u3-u5-u7 sequence:
MVSKGEELFTGVKLTLKFICTTTYKTRAEVKFEGDHNVYITADKQ (SEQ ID NO.: 15)
u8-u9 sequence (A1 is none):
GIKANFKIRHNVEDVQLADHYQQNTPIG (SEQ ID NO.: 14)
A1-u5-EK-u6 sequence:
MVSKGEELFTGVTYKTRAEVKFEGDDDDDKTLVNRIELKGIDF (SEQ ID NO.: 22)
A1-u1-u2-u3 sequence:
MVSKGEELFTGVVPILVELDGDVNGHKFSVRGEGEGDATKLTLKFICTT (SEQ ID NO.: 26)
A1-u1-u2-u3-u4 sequence:

MVSKGEELFTGVVPILVELDGDVNGHKFSVRGEGEGDATKLTLKFICTT
YVQERTISFKD (SEQ ID NO.: 27)

A1-u3-u4-u6 sequence:
MVSKGEELFTGVKLTLKFICTTYVQERTISFKDTLVNRIELKGIDF (SEQ ID NO.: 28)
A1-u4-u7-u10 sequence:

MVSKGEELFTGVYVQERTISFKDHNVYITADKQHYLSTQSVLSKD (SEQ ID NO.: 29)

A1-u3-u4-u7-u10 sequence:

MVSKGEELFTGVKLTLKFICTTYVQERTISFKDHNVYITADKQHYLSTQS

VLSKD (SEQ ID NO.: 30)

In another preferred embodiment, the first linker peptide and/or the second linker peptide have an amino acid sequence as shown in SEQ ID NO.: 18 (ENLYFQGR).

In another preferred embodiment, the proinsulin has the amino acid sequence as shown in SEQ ID NO: 19 or 20.

FVNQHLCGSHLVEALYLVCGERGFFYTPKTRREAEDLQVGQVELGGGPG

AGSLQPLALEGSLQKRGIVEQCCTSICSLYQLENYCN (SEQ ID NO.: 19)

FVNQHLCGSHLVEALYLVCGERGFFYTPKTRGIVEQCCTSICSLYQLENYC

N (SEQ ID NO.: 20)

In another preferred embodiment, the fusion protein has an amino acid sequence as shown in SEQ ID NO.: 21.

MVSKGEELFTGVYVQERTISFKDTYKTRAEVKFEGDENLYFQGRFVNQ

HLCGSHLVEALYLVCGERGFFYTPKTRGIVEQCCTSICSLYQLENYCN    (SEQ

ID NO.: 21)

As used herein, the term "fusion protein" also includes variant forms having the above-mentioned activities. These variant forms include (but are not limited to): 1-3 (usually 1-2, more preferably 1) amino acid deletions, insertions and/or substitutions, and one or several (usually 3 or less, preferably 2 or less, more preferably 1 or less) amino acids are added or deleted at the C-terminal and/or N-terminal. For example, in this field, when amino acids with close or similar properties are substituted, the function of the protein is usually not changed. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above-mentioned fusion protein. As used herein, the terms "fragment", "derivative" and "analog" refer to a polypeptide that substantially retains the function or activity of the fusion protein of the present invention. The polypeptide fragments, derivatives or analogs of the present invention can be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of a polypeptide with another compound (such as a compound that prolongs the half-life of polypeptide, such as polyethylene glycol), or (iv) the polypeptide formed by fusion of additional amino acid sequence to this polypeptide sequence (fusion protein formed by fusion with leader sequence, secretory sequence or 6His tag sequence). According to the teachings herein, these fragments, derivatives and analogs fall within the scope well known to those skilled in the art.

A preferred type of active derivative means that compared with the amino acid sequence of the present invention, at most 3, preferably at most 2, and more preferably at most 1 amino acid are replaced by amino acids with close or similar properties to form a polypeptide. These conservative variant polypeptides are best produced according to Table A by performing amino acid substitutions.

Table A

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion protein of the present invention. The difference between these analogs and the polypeptide of the present invention may be a difference in amino acid sequence, may also be a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides exemplified above.

In addition, the fusion protein of the present invention can also be modified. Modified (usually without changing the primary structure) forms include: chemically derived forms of polypeptides *in vivo* or *in vitro,* such as acetylation or carboxylation. Modifications also include glycosylation, such as those polypeptides produced by glycosylation modifications during the synthesis and processing of the polypeptide or during further processing steps. This modification can be accomplished by exposing the polypeptide to an enzyme that performs glycosylation (such as a mammalian glycosylase or deglycosylase). Modified forms also include sequences with phosphorylated amino acid residues (such as phosphotyrosine, phosphoserine, phosphothreonine). It also includes polypeptides that have been modified to improve their anti-proteolytic properties or optimize their solubility properties.

The term "polynucleotide encoding the fusion protein of the present invention" may include a polynucleotide encoding the fusion protein of the present invention, or a polynucleotide that also includes additional coding and/or non-coding sequences.

The present invention also relates to variants of the above-mentioned polynucleotides, which encode fragments, analogs and derivatives of polypeptides or fusion proteins having the same amino acid sequence as the present invention. These nucleotide variants include substitution variants, deletion variants and insertion variants. As known in the art, an allelic variant is an alternative form of polynucleotide. It may be a substitution, deletion or insertion of one or more nucleotides, but will not substantially change the function of the encoded fusion protein.

The present invention also relates to polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, "strict conditions" refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The fusion protein and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and using a commercially available cDNA library or a cDNA library prepared according to a conventional method known to those skilled in the art as a template to amplify the relevant sequence. When the sequence is long, it is often necessary to perform two or more PCR amplifications, and then each amplified fragments are spliced together in the correct order.

Once the relevant sequence is obtained, the recombination method can be used to obtain the relevant sequence in large quantities. This is usually done by cloning it into a vector, then transferring it into a cell, and then isolating the relevant sequence from the proliferated host cell by conventional methods.

In addition, artificial synthesis methods can also be used to synthesize related sequences, especially when the fragment length is short. Usually, by first synthesizing multiple small fragments, and then ligating to obtain fragments with very long sequences.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used, and the primers used for PCR can be appropriately selected according to the sequence information of the present invention disclosed herein, and can be synthesized by conventional methods. The amplified DNA/RNA fragments can be separated and purified by conventional methods such as gel electrophoresis.

**Expression vector**

The present invention also relates to a vector containing the polynucleotide of the present invention, a host cell produced by genetic engineering using the vector of the present invention or the fusion protein coding sequence of the present invention, and a method for producing the polypeptide of the present invention through recombinant technology.

Through conventional recombinant DNA technology, the polynucleotide sequence of the present invention can be used to express or produce recombinant fusion protein. Generally speaking, there are the following steps:

(1) using the polynucleotide (or variant) of the present invention encoding the fusion protein of the present invention, or using a recombinant expression vector containing the polynucleotide to transform or transduce a suitable host cell;
(2). culturing a host cell in a suitable medium;
(3). isolating and purifying protein from culture medium or cells.

In the present invention, the polynucleotide sequence encoding the fusion protein can be inserted into a recombinant expression vector. The term "recombinant expression vector" refers to bacterial plasmids, bacteriophages, yeast plasmids, plant cell viruses, mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the fusion protein of the present invention and appropriate transcription/translation control signals. These methods include *in vitro* recombinant DNA technology, DNA synthesis technology, and *in vivo* recombination technology. The DNA sequence can be effectively linked to an appropriate promoter in the expression vector to guide mRNA synthesis. Representative examples of these promoters are: Escherichia coli lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase for eukaryotic cell culture, neomycin resistance, and green fluorescent protein (GFP), or tetracycline or ampicillin resistance for *E. coli.*

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: Escherichia coli, Streptomyces; bacterial cells of Salmonella typhimurium; fungal cells such as yeast and plant cells (such as ginseng cells).

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pair SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as Escherichia coli, competent cells that can absorb DNA can be harvested after the exponential growth phase and treated with the $CaCl_2$ method. The steps used are well known in the art. Another method is to use $MgCl_2$. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc..

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are cultured for a period of time.

The recombinant polypeptide in the above method can be expressed in the cell or on the cell membrane, or secreted out of the cell. If necessary, using its physical, chemical and other characteristics to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations of these methods.

**Synthesis of insulin**

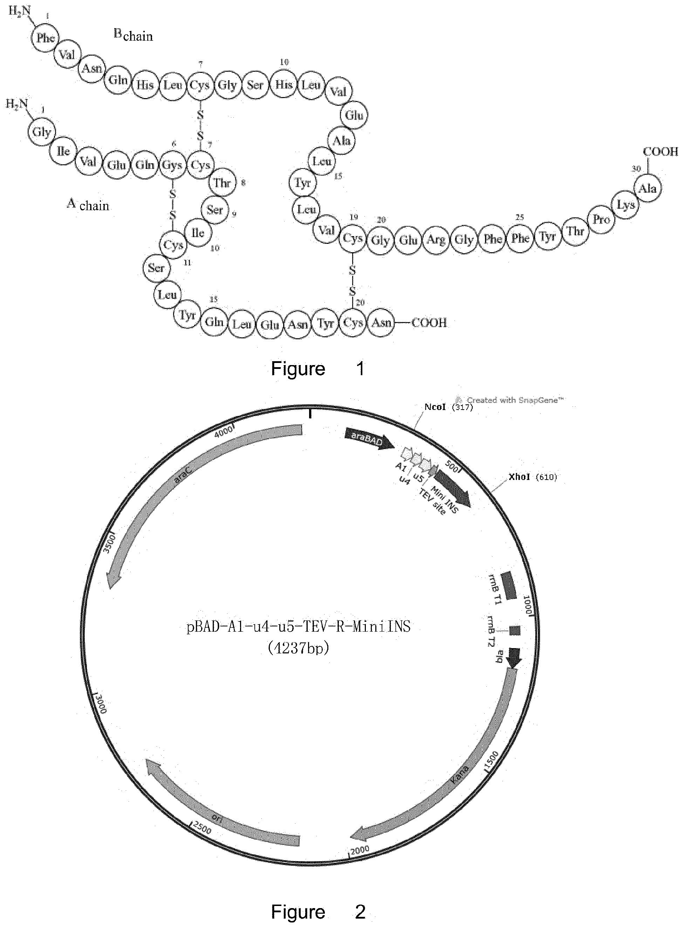

Insulin is a well-defined peptide with known amino acid sequence and structural characteristics. It is a protein with a total of 51 amino acid residues with two amino acid chains. The hormone contains two independent peptide chains, A chain (21 amino acids) and B chain (30 amino acids). There are 6 cysteine residues in the two amino acid chains, and each chain has two cysteine residues connected to each other by disulfide bonds. From a statistical point of view, there are 15 possibilities for disulfide bonds formed in a human insulin molecule. But only one of these 15 possibilities exists in human insulin with biological activity, and its disulfide bonds are as follows: 1) A6-A11; 2) A7-B7; 3) A20-B19. Proinsulin is the biological precursor of insulin. It is a single chain peptide formed by linking A chain and B chain by C peptide. The two peptide chains of insulin are joined by disulfide bonds (Figure 1).

Insulin is a protein hormone secreted by pancreatic islet cells stimulated by endogenous substances such as glucose. The first secreted by pancreatic islet cells is proinsulin, a long-chain polypeptide composed of 84 amino acids. Proinsulin is cleaved off the middle part (C chain) of proinsulin by the action of specific protease-proinsulin converting enzymes PC1 and PC2 and carboxypeptidase E (CPE), while the carboxyl part (A chain) and amino part (B chain) of proinsulin are joined together by disulfide bonds to form insulin. Mature insulin is stored in secretory vesicles in pancreatic islet cells and coordinated with zinc ions to form hexamers. Under external stimulation, insulin is released into the blood along with secretory vesicles and exerts its physiological effects. Patients with type 1 diabetes lose their own blood glucose regulation ability due to the destruction of their own pancreatic islet cells' ability to produce insulin.

Currently, there are two routes for the production of various types of commercial recombinant human insulin-the "chain combination" route and the "proinsulin" route. In the "chain combination" route, the two peptide chains that make up insulin-the A chain and the B chain are synthesized separately through biological recombination, and then the A chain and the B chain are mixed to produce disulfide bonds to generate biologically active human insulin. However, the efficiency of directly mixing two peptide chains to produce biologically active human insulin is relatively low, and the final yield is only about 7%. This method is gradually being replaced by the second route-the "proinsulin" route. For the "proinsulin" route, proinsulin composed of insulin B chain, C chain and A chain is first expressed in *E. coli* or yeast, and then renatured *in vitro* after purification. The renatured proinsulin is then hydrolyzed and digested with trypsin and carboxypeptidase B to obtain human insulin with natural activity. In the "proinsulin" route, trypsin specifically recognizes the lysine and arginine in the protein and cleaves the peptide bond at the C-terminus of the lysine and arginine. Due to conformational reasons, the arginine at position B22 of proinsulin is not hydrolyzed by trypsin. However, trypsin recognizes and hydrolyzes the lysine at position B29 of insulin, thereby inevitably producing an insulin by-product (DesB30-insulin) in which threonine at position B30 is eliminated. In order to reduce the production of DesB30-insulin, the amount of trypsin and reaction time must be strictly controlled. Nevertheless, a certain amount of DesB30-insulin will be produced. Because there is only one threonine difference between DesB30-insulin and insulin, the separation between them is very difficult. Large-scale high performance liquid chromatography is widely used in the industry to separate insulin, but the separation of insulin and DesB30-insulin by this method will generate a large amount of industrial waste, leading to high production costs of existing recombinant human insulin.

The present invention develops a recombinant method for obtaining human insulin with correctly connected cysteine bridges. The method requires fewer steps, so that higher yields of human insulin can be obtained.

A DNA fragment was chemically synthesized, the DNA fragment encoding 2β-TEV-R-MiniINS consisting of the amino acid sequence of SEQ ID NO.: 21. The DNA fragment was cloned into a bacterial expression vector regulated by the araBAD promoter. The expression vector containing 2β-TEV-R-MiniINS was transformed into *E. coli* TopIO strain, and the recombinant cells were cultured in LB medium containing trace elements. The 2β-TEV-R-MiniINS fusion protein was recovered from the inclusion body, and folded under certain conditions, making the disulfide bond formed in the folded 2β-TEV-R-MiniINS fusion protein the same as the disulfide bond in the correctly folded human proinsulin, that is, the formed disulfide bonds are A6-A11, A7-B7 and A20-B19. The separated correctly folded 2β-TEV-R-MiniINS fusion protein was digested with trypsin and carboxypeptidase B to form correctly folded Boc-human insulin. Boc-human insulin was purified to 90% purity by hydrophobic chromatography. The Boc was then deprotected by acid to form correctly folded human insulin, which was then purified by reverse phase HPLC. N-terminal sequence analysis, molecular weight determination, and peptide mapping were used to determine the properties of the pure human insulin thus produced.

**Compared with the prior art, the present invention mainly has the following advantages:**

1) The protein expression promotion element in the fusion protein of the present invention can promote the folding of the fusion protein.
2) The protein expression promotion element of the present invention can improve the solubility of the soluble fusion protein and reduce the intermolecular interaction of the fusion protein, so that the fusion protein can be folded at a high concentration of commercial significance.
3) There is no need for cyanogen bromide cleavage, oxidative sulfite hydrolysis and related purification steps during the process of preparing the target peptide.
4) There is no need to use high concentrations of thiols or hydrophobic adsorption resins during the preparation of the target peptides.
5) Protect the target peptides from intracellular degradation of the microbial host.
6) The target protein has a high specific gravity (increased fusion ratio). The (X)n or A1-(X)n in the fusion protein can be digested into small fragments by protease. Compared with the target protein, the molecular weight has a large difference and is easy to separate.
7) The fusion protein of the present invention can promote the expression of the target peptide, and the expression level and yield of the target peptide are significantly improved.
8) The fusion protein of the present invention is very suitable for expressing target peptides with unnatural amino acids, and can obviously promote the folding of target peptides with unnatural amino acids.

The present invention will be further explained below in conjunction with specific embodiments. It should be understood that these embodiments are only used to illustrate the present invention and not to limit the scope of the present invention. The experimental methods without specific conditions in the following examples are usually based on conventional conditions, such as the conditions described in Sambrook et al., Molecular Cloning: Laboratory Manual (New York: Cold Spring Harbor Laboratory Press, 1989), or according to the conditions suggested by the manufacturer. Unless otherwise specified, percentages and parts are weight percentages and parts by weight.

In the embodiments, taking human insulin as the target peptide as an example, but it is not limited thereto.

**Example 1 Construction of expression vector A1-u4-u5-TEV-R-MiniINS**

The expression construct A1-u4-u5-TEV-R-MiniINS contains a gene encoding human insulin protein, which is fused to the C-terminus of A1-u4-u5. The linker peptide between A1-u4-u5 and the insulin protein MiniINS is the octapeptide Glu-Asn-Leu-Tyr-Phe-Gln-Gly-Arg. The octapeptide can be hydrolyzed by trypsin at the carboxyl end of Arg, and can also be hydrolyzed by TEV protease between Gln-Gly. The DNA sequence of the octapeptide is codon-optimized, which can realize high-level expression of functional protein in *E. coli.*

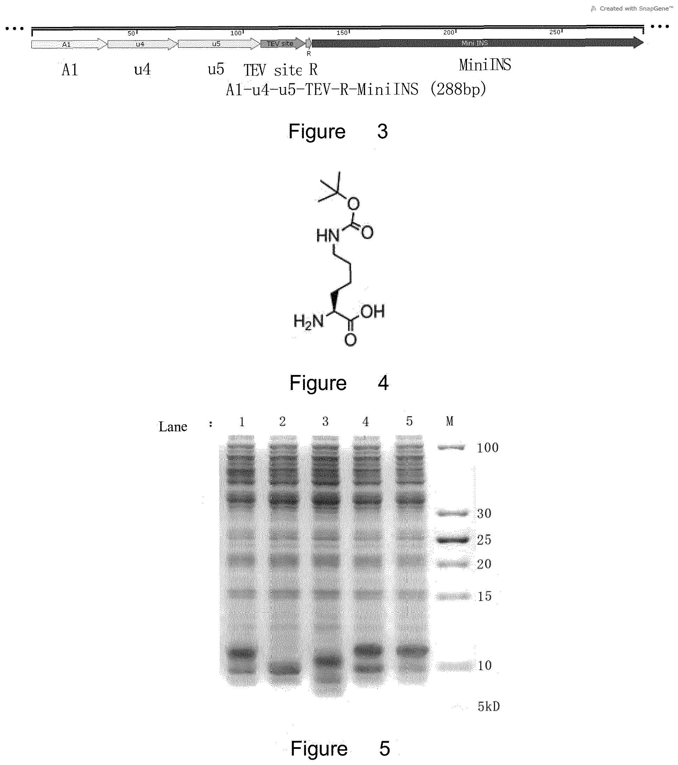

All the fusion recombinant protein fragments used were synthesized by GenScript and loaded into the pUC57 vector. Using restriction enzymes NcoI and XhoI, the "A1-u4-u5-TEV-R-MiniINS" was removed from the synthetic vector "pUC57-A1-u4-u5-TEV-R-MiniINS", and the expression vector "pBAD/His A(KanaR)" was cut with restriction enzymes NcoI and XhoI at the same time. The digested products were separated by agarose electrophoresis, and then using agarose gel DNA recovery kit for extraction, and finally the two DNA fragments were connected using T4 DNA ligase. The ligation product was transformed into *E. coli* Top10 cells, and the transformed cells were cultured on LB agar medium containing 50μg/mL kanamycin (10g/L yeast peptone, 5g/L yeast extract powder, 10g/L NaCl, 1.5% agar) overnight. Picking 3 live colonies and cultured overnight in 5mL liquid LB medium (10g/L yeast peptone, 5g/L yeast extract powder, 10g/L NaCl) containing 50μg/mL kanamycin, and using a Plasmid Mini Extraction Kit for plasmid extraction. Then, the extracted plasmid was sequenced. The finally obtained plasmid was named "pBAD-A1-u4-u5-TEV-R-MiniINS". The plasmid map and the schematic diagram of the fusion protein are shown in Figure 2 and Figure 3, respectively.

**Example 2 Construction of expression construct A1-u4-u5-TEV-R-GLP1**

The pUC57-A1-u4-u5-TEV-R-GLP1 was constructed and synthesized as described in Example 1. At the same time, using restriction enzymes NcoI and XhoI to cut the expression vector "pBAD/His A(KanaR)", and the digested products were separated by agarose electrophoresis, then using agarose gel DNA recovery kit for extraction, and finally using T4 DNA ligase to connect the two DNA fragments. The ligation product was chemically transformed into *E. coli* Top10 cells, and the transformed cells were cultured on LB agar medium containing 50μg/mL kanamycin (10g/L yeast peptone, 5g/L yeast extract powder, 10g/L NaCl, 1.5% agar) overnight. Picking 3 live colonies and cultured overnight in 5mL liquid LB medium (10g/L yeast peptone, 5g/L yeast extract powder, 10g/L NaCl) containing 50μg/mL kanamycin, and using a Plasmid Mini Extraction Kit for plasmid extraction. Then, the extracted plasmid was sequenced using the sequencing oligonucleotide primer 5'-ATGCCATAGCATTTTTATCC-3' to confirm the correct insertion. The resulting plasmid was named "pBAD-A1-u4-u5-TEV-R-GLP1".

**Example 3 Expression, separation and purification of A1-u4-u5-TEV-R-MiniINS fusion protein**

In order to express the fusion fragment of A1-u4-u5-TEV-R-MiniINS containing the amino acid sequence of SEQ ID NO.: 21. The plasmid pBAD-A1-u4-u5-TEV-R-MiniINS confirmed by sequencing and the pyrrolysine aminoacyl tRNA synthetase plasmid pEvol-pylRs-pylT (wherein, the pyrrolysine aminoacyl tRNA synthetase plasmid pEvol-pylRs-pylT was used to express aminoacyl tRNA synthetase and tRNA, as shown in Example 1 of Patent Application No. 2011103886241) and transformed into *E. coli* strain Top1O together. The transformation solution was placed on LB agar medium containing 25 μg/mL kanamycin and 17 μg/mL chloramphenicol overnight. Picking a single colony and cultured overnight in an LB liquid medium containing 25 μg/mL kanamycin and 17 μg/mL chloramphenicol. Then, the overnight culture was inoculated into 100 mL TB medium (Liquid TB medium: 12g/L yeast peptone, 24g/L yeast extract powder, 4mL/L glycerol, 4% KPP, 0.3‰ defoamer. KPP solution: 23.1g/L $KH_2PO4$, 125.4g/L anhydrous $K_2HPO4$) containing 25 μg/mL kanamycin and 17 μg/mL chloramphenicol and incubated at 37° C until $OD_{600}$ is 2-4. Then, 25% arabinose solution was added to the medium to a final concentration of 0.25%, and 0.1M tert-butoxycarbonyl lysine (BOC-lysine, BOC structure as shown in Figure 4) solution was added to a final concentration of 5mM to induce the expression of the fusion protein. The culture solution was cultured continuously for 16-20 hours, and then collected by centrifugation (10000rpm, 5min, 4°C).

The A1-u4-u5-TEV-R-MiniINS fusion protein was expressed in the form of insoluble "inclusion bodies". In order to release the inclusion bodies, the *E. coli* cells were disrupted with a high-pressure homogenizer. Nucleic acids, cell debris and soluble proteins were removed by centrifugation at 10000g. The inclusion bodies containing the A1-u4-u5-TEV-R-MiniINS fusion protein were washed with pure water, and the resulting inclusion body precipitate was used as a raw material for folding. The final expression level of the fusion protein was 14g/L fermentation broth. The SDS-PAGE chart of the fusion protein is shown in Figure 5. It can be seen from Figure 5 that the target protein expressed by the fusion protein can be expressed completely without breakage, and the fusion protein inserted into the unnatural amino acid target protein can also be expressed intact without breakage.

In order to refold the fusion protein, dissolving the inclusion bodies in a 7.5M urea solution pH 10.5 containing 2-10 mM mercaptoethanol so that the total protein concentration after dissolution was 10-25 mg/mL. The sample was diluted 5 to 10 times, and conventional folding was performed for 16 to 30 hours at 4 to 8°C and a pH of 10.5 to 11.7. At 18-25°C, the pH value was maintained at 8.0-9.5, and using trypsin and carboxypeptidase B to digest the fusion protein for 10-20 hours, and then 0.45M ammonium sulfate was added to terminate the enzymatic hydrolysis reaction.

Reversed-phase HPLC analysis results show that the yield of this enzymatic hydrolysis step is higher than 90%. The insulin analog obtained after digestion with trypsin and carboxypeptidase B is named BOC-human insulin. BOC-human insulin cannot be enzymatically hydrolyzed under the above conditions. The sample was clarified by membrane filtration, with 0.45 mM ammonium sulfate as a buffer, and preliminary purification of Boc-human insulin by hydrophobic chromatography. The purity of SDS-polyacrylamide gel electrophoresis reaches 90% and the final yield of BOC-human insulin per 1 liter of fermentation broth is about 2.1 g and the obtained Boc-human insulin was analyzed by MALDI-TOF mass spectrometry, and it is found that its molecular weight is consistent with the theoretical molecular weight of 5907.7 Da. The samples were collected by hydrophobic chromatography, and hydrochloric acid was added to carry out the Boc-human insulin deprotection reaction. Sodium hydroxide solution was added to control the pH to 2.8~3.2 to terminate the reaction. After two steps of high pressure reversed-phase chromatography, the yield of recombinant human insulin is higher than 85%. The final yield of recombinant human insulin per 1 liter of fermentation broth is about 700 mg.

**Example 4 Construction of pBAD-A1-u8-TEV-R-MiniINS expression construct and expression, separation and purification of A1-u8-TEV-R-MiniINS fusion protein**

The experimental method is the same as in Example 1-3, replacing A1-u4-u5 (SEQ ID NO.: 16) in A1-u4-u5-TEV-R-MiniINS with A1-u8 (SEQ ID NO.: 23). The recombinant plasmid named pBAD-A1-u8-TEV-R-MiniINS was constructed, and the A1-u8-TEV-R-MiniINS fusion protein was expressed according to the method (Figure 5). The final yield of BOC-human insulin is about 1.9 g/L and the obtained Boc-human insulin was analyzed by MALDI-TOF mass spectrometry. And the results show that its molecular weight is consistent with the theoretical molecular weight.

**Example 5 Construction of pBAD-A1-u3-u5-u7-TEV-R-MiniINS expression construct and expression, separation and purification of A1-u3-u5-u7-TEV-R-MiniINS fusion protein**

The experimental method is the same as in Example 1-3, replacing A1-u4-u5 (SEQ ID NO.: 16) in A1-u4-u5-TEV-R-MiniINS with A1-u3-u5-u7 (SEQ ID NO. : 15). The recombinant plasmid named pBAD-A1-u3-u5-u7-TEV-R-MiniINS was constructed, and the A1-u3-u5-u7-TEV-R-MiniINS fusion protein was expressed according to the method. The final yield of BOC-human insulin is about 2.0 g/L and the obtained Boc-human insulin was analyzed by MALDI-TOF mass spectrometry. And the results show that its molecular weight is consistent with the theoretical molecular weight.

**Example 6 Construction of pBAD-A1-u5-EK-u6-TEV-R-MiniINS expression construct and expression, separation and purification of A1-u5-EK-u6-TEV-R-MiniINS fusion protein**

The experimental method is the same as in Example 1-3, replacing A1-u4-u5 (SEQ ID NO.: 16) in A1-u4-u5-TEV-R-MiniINS with A1-u5-EK-u6 (SEQ ID NO. : 22), wherein EK is the restriction site of EK enzyme DDDDK (SEQ ID NO.: 25). The recombinant plasmid named pBAD-A1-u5-EK-u6-TEV-R-MiniINS was constructed, and the A1-u5-EK-u6-TEV-R-MiniINS fusion protein was expressed according to the method. The final yield of BOC-human insulin is about 1.9 g/L and the obtained Boc-human insulin was analyzed by MALDI-TOF mass spectrometry. And the results show that its molecular weight is consistent with the theoretical molecular weight.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

**Claims**

1. A fusion protein, comprising the structure as shown in Formula I:

$$(P1\text{-}L1)s\text{-}A1\text{-}(X)n\text{-}A2\text{-}(L2\text{-}P2)t \ (I)$$

wherein

   "-" is a peptide bond or linker peptide,
   each P1 is independently a first target peptide;
   each P2 is independently a second target peptide;
   each L1 is independently none or a first linker peptide;
   each L2 is independently none or a second linker peptide;
   A1 is none or signal peptide;
   A2 is none or signal peptide;
   each X is independently a single β-folding unit of fluorescent protein;
   n is a positive integer from 1-8;
   s is 0, 1, or 2;
   t is 0, 1, or 2; and
   the additional condition is that s and t are not 0 at the same time.

2. The fusion protein of claim 1, wherein the first target peptide and/or the second target peptide are a protein with an unnatural amino acid.

3. The fusion protein of claim 1, wherein the β-folding unit is selected from the group consisting of the β-folding units u1, u2, u3, u4, u5, u6, u7, u8, u9, u10 and u11 of the fluorescent protein.

4. The fusion protein of claim 1, wherein the A1-(X)n has an amino acid sequence as shown in SEQ ID NO.: 14, 15, 16, 17, 22, 23, 24, 26, 27, 28, 29 or 30 .

5. The fusion protein of claim 1, wherein there is a flexible linker I between any two β-folding units, and the flexible linker I contains a third restriction site.

6. An isolated oligonucleotide encoding the fusion protein of claim 1.

7. A vector, which includes the oligonucleotide of claim 6.

8. A host cell containing the vector of claim 7, or the exogenous oligonucleotide of claim 6 integrated into the chromosome, or expresses the fusion protein of claim 1.

9. A method for preparing a protein, comprising the steps:

    (a) culturing the host cell of claim 8, thereby obtaining the fusion protein of claim 1.

10. Use of the fusion protein of claim 1, or the oligonucleotide of claim 6, or the vector of claim 7, or the host cell of claim 8 for expression and preparation of the target polypeptide.

Figure 1

Figure 2

A1     u4     u5  TEV site R       MiniINS

A1-u4-u5-TEV-R-MiniINS (288bp)

Figure    3

Figure    4

Figure    5

## INTERNATIONAL SEARCH REPORT

| | International application No. |
|---|---|
| | **PCT/CN2020/080036** |

**A. CLASSIFICATION OF SUBJECT MATTER**

C07K 19/00(2006.01)i; C12N 15/11(2006.01)i; C12N 15/63(2006.01)i; C12N 15/00(2006.01)i

According to International Patent Classification (IPC) or to both national classification and IPC

**B. FIELDS SEARCHED**

Minimum documentation searched (classification system followed by classification symbols)

C07K，C12N

Documentation searched other than minimum documentation to the extent that such documents are included in the fields searched

Electronic data base consulted during the international search (name of data base and, where practicable, search terms used)

CNABS, DWPI, SIPOABS, CNKI, 万方, WANFANG, ISI WEB OF KNOWLEDGE, BAIDU and search items: 融合蛋白, 肽, 胰岛素, 荧光蛋白, GFP, YFP, BFP, CFP, β-折叠, 二硫键, fusion protein, peptide, insulin, GLP1, fluorescent protein, β-sheet, disulfide bond, 2β-TEV-R-MiniINS; Genbank, EMBL, 中国专利生物序列检索系统, STN和检索的序列: SEQ ID NOs: 1-30, GENBANK, EMBL, CHINA PATENT BIOLOGICAL SEQUENCE SEARCH SYSTEM, STN and searched sequences: SEQ ID NOs: 1-30.

**C. DOCUMENTS CONSIDERED TO BE RELEVANT**

| Category* | Citation of document, with indication, where appropriate, of the relevant passages | Relevant to claim No. |
|---|---|---|
| A | CN 102348715 A (AMUNIX OPERATING INC.) 08 February 2012 (2012-02-08) entire document | 1-10 |
| A | WO 2017094885 A1 (UNIV TOKYO et al.) 08 June 2017 (2017-06-08) entire document | 1-10 |
| A | WO 2011123813 A2 (AMUNIX OPERATING INC. et al.) 06 October 2011 (2011-10-06) entire document | 1-10 |
| A | CN 108474021 A (DUKE UNIVERSITY) 31 August 2018 (2018-08-31) entire document | 1-10 |

☐ Further documents are listed in the continuation of Box C.    ☑ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **07 May 2020** | **17 June 2020** |

| Name and mailing address of the ISA/CN | Authorized officer |
|---|---|
| **China National Intellectual Property Administration** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/080036**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
Information on patent family members

International application No.

**PCT/CN2020/080036**

| Patent document cited in search report | | | Publication date (day/month/year) | Patent family member(s) | | | Publication date (day/month/year) |
|---|---|---|---|---|---|---|---|
| CN | 102348715 | A | 08 February 2012 | NZ | 593833 | A | 25 October 2013 |
| | | | | NZ | 606427 | A | 31 October 2014 |
| | | | | US | 9371369 | B2 | 21 June 2016 |
| | | | | EA | 201170993 | A1 | 28 February 2012 |
| | | | | EA | 020843 | B1 | 27 February 2015 |
| | | | | ZA | 201407665 | B | 19 December 2018 |
| | | | | HR | P20161807 | T1 | 24 February 2017 |
| | | | | BR | PI1008061 | A2 | 15 March 2016 |
| | | | | IL | 213562 | D0 | 31 July 2011 |
| | | | | EP | 3581579 | A1 | 18 December 2019 |
| | | | | AU | 2010210618 | B2 | 21 August 2014 |
| | | | | US | 2010239554 | A1 | 23 September 2010 |
| | | | | ZA | 201104758 | B | 27 May 2015 |
| | | | | US | 2017037088 | A1 | 09 February 2017 |
| | | | | HR | P20191020 | T1 | 06 September 2019 |
| | | | | WO | 2010091122 | A1 | 12 August 2010 |
| | | | | JP | 6265564 | B2 | 24 January 2018 |
| | | | | CN | 102348715 | B | 08 December 2017 |
| | | | | EP | 2393828 | B1 | 12 October 2016 |
| | | | | MX | 2011008094 | A | 13 February 2012 |
| | | | | US | 2014301974 | A1 | 09 October 2014 |
| | | | | JP | 2017031218 | A | 09 February 2017 |
| | | | | JP | 2019213548 | A | 19 December 2019 |
| | | | | CA | 2748314 | C | 02 October 2018 |
| | | | | IL | 245994 | D0 | 31 July 2016 |
| | | | | EP | 3178835 | A1 | 14 June 2017 |
| | | | | HU | E031840 | T2 | 28 August 2017 |
| | | | | LT | 2393828 | T | 25 January 2017 |
| | | | | US | 9926351 | B2 | 27 March 2018 |
| | | | | AU | 2010210618 | A1 | 21 July 2011 |
| | | | | SI | EP2393828 | T1 | 31 January 2017 |
| | | | | BR | PI1008061 | A8 | 04 October 2016 |
| | | | | PL | 2393828 | T3 | 30 June 2017 |
| | | | | JP | 2018076378 | A | 17 May 2018 |
| | | | | JP | 6012684 | B2 | 25 October 2016 |
| | | | | DK | 3178835 | T3 | 24 June 2019 |
| | | | | US | 2018244736 | A1 | 30 August 2018 |
| | | | | CN | 108530543 | A | 14 September 2018 |
| | | | | IL | 213562 | A | 31 January 2019 |
| | | | | EP | 2393828 | A1 | 14 December 2011 |
| | | | | MX | 362028 | B | 04 January 2019 |
| | | | | KR | 101544245 | B1 | 12 August 2015 |
| | | | | JP | 2016106135 | A | 16 June 2016 |
| | | | | JP | 6348946 | B2 | 27 June 2018 |
| | | | | NZ | 628987 | A | 27 November 2015 |
| | | | | DK | 2393828 | T3 | 23 January 2017 |
| | | | | PT | 2393828 | T | 18 January 2017 |
| | | | | PT | 3178835 | T | 19 June 2019 |
| | | | | EP | 3178835 | B1 | 10 April 2019 |
| WO | 2017094885 | A1 | 08 June 2017 | JP | WO2017094885 | A1 | 20 September 2018 |

Form PCT/ISA/210 (patent family annex) (January 2015)

**INTERNATIONAL SEARCH REPORT**
Information on patent family members

International application No.

**PCT/CN2020/080036**

| Patent document cited in search report | | | Publication date (day/month/year) | Patent family member(s) | | | Publication date (day/month/year) |
|---|---|---|---|---|---|---|---|
| WO | 2011123813 | A2 | 06 October 2011 | EP | 3372617 | A2 | 12 September 2018 |
| | | | | EP | 2552967 | A2 | 06 February 2013 |
| | | | | EP | 2552967 | A4 | 08 October 2014 |
| | | | | WO | 2011123813 | A3 | 01 December 2011 |
| CN | 108474021 | A | 31 August 2018 | CA | 3005831 | A1 | 26 May 2017 |
| | | | | US | 2018328935 | A1 | 15 November 2018 |
| | | | | EP | 3377643 | A1 | 26 September 2018 |
| | | | | EP | 3377643 | A4 | 02 October 2019 |
| | | | | AU | 2016357091 | A1 | 07 June 2018 |
| | | | | WO | 2017087051 | A1 | 26 May 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011103886241 A

**Non-patent literature cited in the description**

- **SAMBROOK et al.** Molecular Cloning: Laboratory Manual. Cold Spring Harbor Laboratory Press, 1989

## Sequence listing

<110> NINGBO KUNPENG BIOTECH CO., LTD.

<120>   FUSION PROTEIN CONTAINING FLUORESCENT PROTEIN FRAGMENTS AND USES THEREOF

<130>   P2020-0086

<150>   CN201910210102.9
<151>   2019-03-19

<160>   31

<170>   PatentIn version 3.5

<210>   1
<211>   13
<212>   PRT
<213> artificial sequence

<220>
<223> u1

<400>   1

Val Pro Ile Leu Val Glu Leu Asp Gly Asp Val Asn Gly
1               5                   10


<210>   2
<211>   14
<212>   PRT
<213> artificial sequence

<220>
<223> u2

<400>   2

His Lys Phe Ser Val Arg Gly Glu Gly Glu Gly Asp Ala Thr
1               5                   10


<210>   3
<211>   10
<212>   PRT
<213> artificial sequence

<220>
<223> u3

<400>   3

Lys Leu Thr Leu Lys Phe Ile Cys Thr Thr
1               5                   10


<210>   4
<211>   11
<212>   PRT
<213> artificial sequence

<220>
<223> u4

<400>    4

Tyr Val Gln Glu Arg Thr Ile Ser Phe Lys Asp
1               5                   10


<210>    5
<211>    13
<212>    PRT
<213> artificial sequence

<220>
<223> u5

<400>    5

Thr Tyr Lys Thr Arg Ala Glu Val Lys Phe Glu Gly Asp
1               5                   10


<210>    6
<211>    13
<212>    PRT
<213> artificial sequence

<220>
<223> u6

<400>    6

Thr Leu Val Asn Arg Ile Glu Leu Lys Gly Ile Asp Phe
1               5                   10


<210>    7
<211>    10
<212>    PRT
<213> artificial sequence

<220>
<223> u7

<400>    7

His Asn Val Tyr Ile Thr Ala Asp Lys Gln
1               5                   10


<210>    8
<211>    14
<212>    PRT
<213> artificial sequence

<220>
<223> u8

<400>    8

Gly Ile Lys Ala Asn Phe Lys Ile Arg His Asn Val Glu Asp
1               5                   10

<210> 9
<211> 14
<212> PRT
<213> artificial sequence

<220>
<223> u9

<400> 9

Val Gln Leu Ala Asp His Tyr Gln Gln Asn Thr Pro Ile Gly
1               5                   10


<210> 10
<211> 12
<212> PRT
<213> artificial sequence

<220>
<223> u10

<400> 10

His Tyr Leu Ser Thr Gln Ser Val Leu Ser Lys Asp
1               5                   10


<210> 11
<211> 13
<212> PRT
<213> artificial sequence

<220>
<223> u11

<400> 11

His Met Val Leu Leu Glu Phe Val Thr Ala Ala Gly Ile
1               5                   10


<210> 12
<211> 12
<212> PRT
<213> artificial sequence

<220>
<223>  A1 or A2

<400> 12

Met Val Ser Lys Gly Glu Glu Leu Phe Thr Gly Val
1               5                   10


<210> 13
<211> 241
<212> PRT
<213> artificial sequence

<220>
<223>   GFP

<400>   13

| Met | Val | Ser | Lys | Gly | Glu | Glu | Leu | Phe | Thr | Gly | Val | Val | Pro | Ile | Leu |
| 1 | | | | 5 | | | | | 10 | | | | | 15 | |

| Val | Glu | Leu | Asp | Gly | Asp | Val | Asn | Gly | His | Lys | Phe | Ser | Val | Arg | Gly |
| | | | 20 | | | | | 25 | | | | | 30 | | |

| Glu | Gly | Glu | Gly | Asp | Ala | Thr | Asn | Gly | Lys | Leu | Thr | Leu | Lys | Phe | Ile |
| | | 35 | | | | | 40 | | | | | 45 | | | |

| Cys | Thr | Thr | Gly | Lys | Leu | Pro | Val | Pro | Trp | Pro | Thr | Leu | Val | Thr | Thr |
| | 50 | | | | | 55 | | | | | 60 | | | | |

| Leu | Thr | Tyr | Gly | Val | Gln | Cys | Phe | Ser | Arg | Tyr | Pro | Asp | His | Met | Lys |
| 65 | | | | | 70 | | | | | 75 | | | | | 80 |

| Arg | His | Asp | Phe | Phe | Lys | Ser | Ala | Met | Pro | Glu | Gly | Tyr | Val | Gln | Glu |
| | | | | 85 | | | | | 90 | | | | | 95 | |

| Arg | Thr | Ile | Ser | Phe | Lys | Asp | Asp | Gly | Thr | Tyr | Lys | Thr | Arg | Ala | Glu |
| | | | 100 | | | | | 105 | | | | | 110 | | |

| Val | Lys | Phe | Glu | Gly | Asp | Thr | Leu | Val | Asn | Arg | Ile | Glu | Leu | Lys | Gly |
| | | 115 | | | | | 120 | | | | | 125 | | | |

| Ile | Asp | Phe | Lys | Glu | Asp | Gly | Asn | Ile | Leu | Gly | His | Lys | Leu | Glu | Tyr |
| | 130 | | | | | 135 | | | | | 140 | | | | |

| Asn | Phe | Asn | Ser | His | Asn | Val | Tyr | Ile | Thr | Ala | Asp | Lys | Gln | Lys | Asn |
| 145 | | | | | 150 | | | | | 155 | | | | | 160 |

| Gly | Ile | Lys | Ala | Asn | Phe | Lys | Ile | Arg | His | Asn | Val | Glu | Asp | Gly | Ser |
| | | | | 165 | | | | | 170 | | | | | 175 | |

| Val | Gln | Leu | Ala | Asp | His | Tyr | Gln | Gln | Asn | Thr | Pro | Ile | Gly | Asp | Gly |
| | | | 180 | | | | | 185 | | | | | 190 | | |

| Pro | Val | Leu | Leu | Pro | Asp | Asn | His | Tyr | Leu | Ser | Thr | Gln | Ser | Val | Leu |
| | | 195 | | | | | 200 | | | | | 205 | | | |

| Ser | Lys | Asp | Pro | Asn | Glu | Lys | Arg | Asp | His | Met | Val | Leu | Leu | Glu | Phe |
| | 210 | | | | | 215 | | | | | 220 | | | | |

| Val | Thr | Ala | Ala | Gly | Ile | Thr | His | Gly | Met | Asp | Glu | Leu | Tyr | Ala | Gly |
| 225 | | | | | 230 | | | | | 235 | | | | | 240 |

Ser

<210> 14
<211> 28
<212> PRT
<213> artificial sequence

<220>
<223> u8-u9

<400> 14

Gly Ile Lys Ala Asn Phe Lys Ile Arg His Asn Val Glu Asp Val Gln
1               5                   10                  15


Leu Ala Asp His Tyr Gln Gln Asn Thr Pro Ile Gly
            20                  25


<210> 15
<211> 45
<212> PRT
<213> artificial sequence

<220>
<223> A1-u3-u5-u7

<400> 15

Met Val Ser Lys Gly Glu Glu Leu Phe Thr Gly Val Lys Leu Thr Leu
1               5                   10                  15


Lys Phe Ile Cys Thr Thr Thr Tyr Lys Thr Arg Ala Glu Val Lys Phe
            20                  25                  30


Glu Gly Asp His Asn Val Tyr Ile Thr Ala Asp Lys Gln
            35                  40                  45


<210> 16
<211> 36
<212> PRT
<213> artificial sequence

<220>
<223>   A1-u4-u5

<400> 16

Met Val Ser Lys Gly Glu Glu Leu Phe Thr Gly Val Tyr Val Gln Glu
1               5                   10                  15


Arg Thr Ile Ser Phe Lys Asp Thr Tyr Lys Thr Arg Ala Glu Val Lys
            20                  25                  30

```
Phe Glu Gly Asp
            35


<210>  17
<211>  26
<212>  PRT
<213> artificial sequence

<220>
<223>   A1-u9

<400>  17

Met Val Ser Lys Gly Glu Glu Leu Phe Thr Gly Val Val Gln Leu Ala
1               5                   10                  15


Asp His Tyr Gln Gln Asn Thr Pro Ile Gly
            20                  25


<210>  18
<211>  8
<212>  PRT
<213> artificial sequence

<220>
<223>   the first linker peptide and/or the second linker peptide

<400>  18

Glu Asn Leu Tyr Phe Gln Gly Arg
1               5


<210>  19
<211>  86
<212>  PRT
<213> artificial sequence

<220>
<223>   the proinsulin

<400>  19

Phe Val Asn Gln His Leu Cys Gly Ser His Leu Val Glu Ala Leu Tyr
1               5                   10                  15


Leu Val Cys Gly Glu Arg Gly Phe Phe Tyr Thr Pro Lys Thr Arg Arg
            20                  25                  30


Glu Ala Glu Asp Leu Gln Val Gly Gln Val Glu Leu Gly Gly Gly Pro
            35                  40                  45


Gly Ala Gly Ser Leu Gln Pro Leu Ala Leu Glu Gly Ser Leu Gln Lys
        50                  55                  60
```

Arg Gly Ile Val Glu Gln Cys Cys Thr Ser Ile Cys Ser Leu Tyr Gln
65                  70              75                  80

Leu Glu Asn Tyr Cys Asn
                85

<210> 20
<211> 52
<212> PRT
<213> artificial sequence

<220>
<223>    the proinsulin

<400> 20

Phe Val Asn Gln His Leu Cys Gly Ser His Leu Val Glu Ala Leu Tyr
1               5               10              15

Leu Val Cys Gly Glu Arg Gly Phe Phe Tyr Thr Pro Lys Thr Arg Gly
            20              25              30

Ile Val Glu Gln Cys Cys Thr Ser Ile Cys Ser Leu Tyr Gln Leu Glu
        35              40              45

Asn Tyr Cys Asn
        50

<210> 21
<211> 96
<212> PRT
<213> artificial sequence

<220>
<223>    fusion protein

<400> 21

Met Val Ser Lys Gly Glu Glu Leu Phe Thr Gly Val Tyr Val Gln Glu
1               5               10              15

Arg Thr Ile Ser Phe Lys Asp Thr Tyr Lys Thr Arg Ala Glu Val Lys
            20              25              30

Phe Glu Gly Asp Glu Asn Leu Tyr Phe Gln Gly Arg Phe Val Asn Gln
        35              40              45

His Leu Cys Gly Ser His Leu Val Glu Ala Leu Tyr Leu Val Cys Gly
        50              55              60

Glu Arg Gly Phe Phe Tyr Thr Pro Lys Thr Arg Gly Ile Val Glu Gln
65                  70              75                  80

```
Cys Cys Thr Ser Ile Cys Ser Leu Tyr Gln Leu Glu Asn Tyr Cys Asn
                85                  90                  95
```

```
<210>   22
<211>   43
<212>   PRT
<213> artificial sequence

<220>
<223>   A1-u5-EK-u6

<400>   22
```

```
Met Val Ser Lys Gly Glu Glu Leu Phe Thr Gly Val Thr Tyr Lys Thr
1               5                   10                  15


Arg Ala Glu Val Lys Phe Glu Gly Asp Asp Asp Asp Lys Thr Leu
                20                  25                  30


Val Asn Arg Ile Glu Leu Lys Gly Ile Asp Phe
        35                  40
```

```
<210>   23
<211>   26
<212>   PRT
<213> artificial sequence

<220>
<223>   A1-u8

<400>   23
```

```
Met Val Ser Lys Gly Glu Glu Leu Phe Thr Gly Val Gly Ile Lys Ala
1               5                   10                  15


Asn Phe Lys Ile Arg His Asn Val Glu Asp
                20                  25
```

```
<210>   24
<211>   36
<212>   PRT
<213> artificial sequence

<220>
<223>   A1-u2-u3

<400>   24
```

```
Met Val Ser Lys Gly Glu Glu Leu Phe Thr Gly Val His Lys Phe Ser
1               5                   10                  15


Val Arg Gly Glu Gly Glu Gly Asp Ala Thr Lys Leu Thr Leu Lys Phe
            20                  25                  30
```

22

```
Ile Cys Thr Thr
        35


<210>  25
<211>  5
<212>  PRT
<213> artificial sequence

<220>
<223>   the restriction site of EK enzyme

<400>  25

Asp Asp Asp Asp Lys
1               5

<210>  26
<211>  49
<212>  PRT
<213> artificial sequence

<220>
<223>   A1-u1-u2-u3

<400>  26

Met Val Ser Lys Gly Glu Glu Leu Phe Thr Gly Val Val Pro Ile Leu
1               5                   10                  15


Val Glu Leu Asp Gly Asp Val Asn Gly His Lys Phe Ser Val Arg Gly
                20                  25                  30


Glu Gly Glu Gly Asp Ala Thr Lys Leu Thr Leu Lys Phe Ile Cys Thr
        35                  40                  45


Thr


<210>  27
<211>  60
<212>  PRT
<213> artificial sequence

<220>
<223>   A1-u1-u2-u3-u4

<400>  27

Met Val Ser Lys Gly Glu Glu Leu Phe Thr Gly Val Val Pro Ile Leu
1               5                   10                  15


Val Glu Leu Asp Gly Asp Val Asn Gly His Lys Phe Ser Val Arg Gly
                20                  25                  30


Glu Gly Glu Gly Asp Ala Thr Lys Leu Thr Leu Lys Phe Ile Cys Thr
        35                  40                  45
```

23

```
Thr Tyr Val Gln Glu Arg Thr Ile Ser Phe Lys Asp
    50                  55                  60


<210>  28
<211>  46
<212>  PRT
<213> artificial sequence

<220>
<223>    A1-u3-u4-u6

<400>  28

Met Val Ser Lys Gly Glu Glu Leu Phe Thr Gly Val Lys Leu Thr Leu
1               5                   10                  15


Lys Phe Ile Cys Thr Thr Tyr Val Gln Glu Arg Thr Ile Ser Phe Lys
            20                  25                  30


Asp Thr Leu Val Asn Arg Ile Glu Leu Lys Gly Ile Asp Phe
        35                  40                  45


<210>  29
<211>  45
<212>  PRT
<213> artificial sequence

<220>
<223>    A1-u4-u7-u10

<400>  29

Met Val Ser Lys Gly Glu Glu Leu Phe Thr Gly Val Tyr Val Gln Glu
1               5                   10                  15


Arg Thr Ile Ser Phe Lys Asp His Asn Val Tyr Ile Thr Ala Asp Lys
            20                  25                  30


Gln His Tyr Leu Ser Thr Gln Ser Val Leu Ser Lys Asp
        35                  40                  45


<210>  30
<211>  55
<212>  PRT
<213> artificial sequence

<220>
<223>    A1-u3-u4-u7-u10

<400>  30

Met Val Ser Lys Gly Glu Glu Leu Phe Thr Gly Val Lys Leu Thr Leu
1               5                   10                  15
```

```
Lys Phe Ile Cys Thr Thr Tyr Val Gln Glu Arg Thr Ile Ser Phe Lys
         20                  25                  30
```

```
Asp His Asn Val Tyr Ile Thr Ala Asp Lys Gln His Tyr Leu Ser Thr
         35                  40                  45
```

```
Gln Ser Val Leu Ser Lys Asp
     50                  55
```

```
<210>   31
<211>   20
<212>   DNA
<213> artificial sequence

<220>
<223>     Oligonucleosides

<400>   31
atgccatagc atttttatcc                                          20
```